# EUROPÄISCHE PATENTSCHRIFT

(11) **EP 1 677 717 B1**
(45) Veröffentlichungstag und Bekanntmachung des Hinweises auf die Patenterteilung: **27.02.2013**
(21) Anmeldenummer: 04790728.2
(22) Anmeldetag: 21.10.2004
(51) Int. Cl.: A61F 9/009, B23K 26/04

(54) **LASERBEARBEITUNG**
LASER MACHINING
USINAGE AU LASER

(30) Priorität: 23.10.2003 DE 10349296; 23.10.2003 DE 10349297
(43) Veröffentlichungstag der Anmeldung: 12.07.2006
(73) Patentinhaber: Carl Zeiss Meditec AG, 07745 Jena (DE)
(72) Erfinder: BISCHOFF, Mark, 07749 Jena (DE); EBERT, Elke, 07743 Jena (DE); FESTAG, Karsten, 07749 Jena (DE); LANG, Carsten, 07607 EISENBERG (DE); STICKER, Markus, 07743 Jena (DE)
(74) Vertreter: Geyer, Fehners & Partner
(86) Internationale Anmeldenummer: PCT/EP2004/011928
(87) Internationale Veröffentlichungsnummer: WO 2005/039462

(56) Entgegenhaltungen:
- EP-A- 0 608 052
- EP-A- 1 159 986
- WO-A-03/002008
- US-A- 5 645 550
- US-B1- 6 373 571

## Beschreibung

Die Erfindung bezieht sich auf einen Adapter zum Koppeln einer Laserbearbeitungsvorrichtung mit einem zu bearbeitenden Objekt, wobei der Adapter eine Eingangsseite aufweist, die über einen Verschlußmechanismus gegenüber der Laserbearbeitungsvorrichtung fixierbar ist, zur Ausrichtung des Objektes gegenüber der Laserbearbeitungsvorrichtung am Objekt befestigbar ist, einen von der Laserbearbeitungsvorrichtung über einen gewissen Bereich gescannt an der Eingangsseite zugeführten Laserstrahl über einen Strahlengang zum Objekt leitet und eine Referenzstruktur aufweist. Die Erfindung bezieht sich weiter auf eine Laserbearbeitungsvorrichtung für einen solchen Adapter mit einer Strahlablenkungseinrichtung zum Scannen eines Laserstrahls.

Bei der Materialbearbeitung mittels Laserstrahlung wird meist ein Abrastern der zu bearbeitenden Gebiete des Objektes mit dem Laserstrahl eingesetzt. Die Genauigkeit der Positionierung des Laserstrahls bestimmt dabei in der Regel die bei der Bearbeitung erzielte Präzision. Wird der Laserstrahl in ein Bearbeitungsvolumen fokussiert, bedarf es einer exakten dreidimensionalen Positionierung. Für eine hochgenaue Bearbeitung ist es deshalb in der Regel unerläßlich, das Objekt in exakt definierter Lage zur Laserbearbeitungsvorrichtung zu halten. Für solche Anwendungen dient der eingangs genannte Adapter, da er das zu bearbeitende Objekt fixiert bzw. der verformbaren Oberfläche des zu bearbeitenden Objektes eine gewünschte Soll-Form verleiht.

Dies ist insbesondere bei der Mikrobearbeitung von Materialien notwendig, die nur eine geringe lineare optische Absorption im Spektralbereich der bearbeitenden Laserstrahlung aufweisen. Bei solchen Materialien werden üblicherweise nicht-lineare Wechselwirkungen zwischen Laserstrahlung und Material eingesetzt, meist in Form eines optischen Durchbruches, der im Fokus des Laserstrahls erzeugt wird. Da die bearbeitende Wirkung nur im Laserstrahlfokus stattfindet, ist es unerläßlich, den Fokuspunkt exakt dreidimensional auszurichten. Zusätzlich zu einer zweidimensionalen Ablenkung des Laserstrahls ist somit eine exakte Tiefenstellung der Fokuslage erforderlich. Der eingangs genannte Adapter erleichtert dies, da er konstante und auch mit einer gewissen Genauigkeit bekannte optische Verhältnisse im Strahlengang zum Objekt sicherstellt bzw. bei an dem Objekt anliegendem Adapter bekannte optische Verhältnisse im Strahlengang insbesondere Brechungsverhältnisse vorliegen.

Eine typische Anwendung für einen solchen Adapter ist das als LASIK bekannte augenoptische Operationsverfahren, bei dem ein Laserstrahl auf einen Fokuspunkt in der Größenordnung eines Mikrometers in die Hornhaut fokussiert wird. Im Fokus entsteht dann ein Plasma, das eine lokale Trennung des Hornhautgewebes bewirkt. Durch geeignete Aneinanderreihung der auf diese Weise erzeugten lokalen Trennungszonen werden makroskopische Schnitte realisiert und ein bestimmtes Hornhautteilvolumen isoliert. Durch Entnahme des Teilvolumens wird dann eine gewünschte Brechungsänderung der Hornhaut erreicht, so daß eine Fehlsichtigkeitskorrektur möglich ist.

Zur Ausführung des Verfahrens ist die exakte Positionierung des Laserstrahls unerläßlich. Dazu ist aus der US 6.373.571 eine mit Referenzmarken versehene Kontaktlinse bekannt, die einen Adapter der eingangs genannten Art realisiert. Diese Kontaktlinse wird mittels einer separaten Meßvorrichtung einjustiert, wodurch ein relativ aufwendiger Aufbau bedingt ist. Der erwähnte Adapter erfüllt zwei Funktionen: er stellt nicht nur die erforderlichen optischen Eigenschaften bei der Einbringung des Laserstrahls in die Hornhaut sicher, sondern er fixiert das Auge auch, vorzugsweise bezüglich mehrerer Freiheitsgrade, besonders bevorzugt bezüglich aller möglichen Freiheitsgrade. Bewegungen des Auges relativ zur Laserbearbeitungsvorrichtung werden damit verhindert.

Ein Beispiel für einen solchen Adapter ist beispielsweise in der WO 03/002008 A1 beschrieben. Der dort als "Planarisierungslinse" bezeichnete Adapter weist einen Saugring auf, der mittels Unterdruck am Auge befestigt ist. In den Saugring ist eine Glasplatte eingelegt, die mittels einer Klammer in den Saugring gepreßt wird. Die Klammer befestigt dabei zugleich ein Flanschteil am Saugring, welches an der Laserbearbeitungsvorrichtung fest angebracht ist. Der mehrteilige Adapter der WO 03/002008 A1 preßt die Oberfläche der Augenhornhaut flach, wodurch einfache Standardgeometrien erreicht werden. Allerdings ist dies für den Patienten sehr unangenehm. Darüber hinaus ist eine Planarisierung der Augenhornhautoberfläche bei manchen chirurgischen Eingriffen unerwünscht.

Ein weiteres Beispiel für einen Adapter der genannten Art ist in der EP 1 159 986 A2 beschrieben. Er weist am Rand einer Halterung Strichmarken auf, die dem Chirurgen eine visuelle Ausrichtung ermöglichen. Die damit erzielte Genauigkeit reicht aber nicht immer aus. Der Erfindung liegt die Aufgabe zugrunde, einen Adapter bzw. eine Laserbearbeitungsvorrichtung der eingangs genannten Art derart weiterzubilden, daß nicht zwingend mit einer planen Geometrie gearbeitet werden muß.

Diese Aufgabe wird gemäß der Erfindung mit einem Adapter zum Koppeln einer Laserbearbeitungsvorrichtung mit einem zu bearbeitenden Objekt, das eine verformbare Oberfläche aufweist, wobei der Adapter eine Eingangsseite aufweist, die über einen Verschlußmechanismus gegenüber der Laserbearbeitungsvorrichtung fixierbar ist, eine Ausgangsseite aufweist, die an die verformbare Oberfläche anlegbar ist und dieser dabei eine gewünschte Soll-Form gibt, wobei der Adapter am Objekt befestigbar ist, einen von der Laserbearbeitungsvorrichtung über einen gewissen Bereich gescannt an der Eingangsseite zugeführten Laserstrahl über einen Strahlengang zur an der Ausgangsseite anliegenden Oberfläche leitet, gelöst, indem der Adapter im Strahlengang Markierungsstrukturen aufweist, die mittels der über den Bereich gescannten Laserstrahlung optisch detektierbar sind und die eine den Adapter kennzeichnende Information codieren.

Die Aufgabe wird weiter mit einer Laserbearbeitungsvorrichtung für einen solchen Adapter, mit einer Strahlablenkungseinrichtung zum Scannen eines Laserstrahls gelöst, die aufweist eine Detektoreinrichtung zum optischen Detektieren der Markierungsstrukturen mittels des Laserstrahls und eine die Detektoreinrichtung auslesende Steuereinrichtung, welche die Strahlablenkungseinrichtung ansteuert, die den Adapter kennzeichnende Information ermittelt und diese bei der Ansteuerung der Strahlablenkungseinrichtung berücksichtigt. Die zuvor hinsichtlich der Justierung bzw. Lagebestimmung des Adapters eingesetzte Referenzstruktur ist also jetzt als Markierungsstruktur zur Informationscodierung ausgebildet. Natürlich ist auch eine Kombination möglich.

Der Adapter dient in der Regel dazu, um eingangsseitig eine feste Kopplung mit der Laserbearbeitungsvorrichtung herzustellen. Die zur Laserbearbeitungsvorrichtung orientierte Eingangsseite des Adapters ist deshalb mit geeigneten Mitteln zur festen Verbindung mit dem zum Objekt orientierten Ausgang (z.B. distalen Ende) der Laserbearbeitungsvorrichtung bzw. deren optischen System ausgebildet, so daß eine auf die Laserbearbeitungsvorrichtung bezogen feste Fixierung mittels eines Verschlußmechanismusses möglich ist. Für den Verschlußmechanismus kommt dabei beispielsweise die Ausbildung einer Flanschfläche am Adapter in Frage.

Ausgangsseitig sorgt der Adapter dafür, daß die verformbare Oberfläche des Objektes eine gewünschte Soll-Form hat. Zur Befestigung des Adapters am Objekt sind geeignete Mittel vorgesehen; bei einer augenchirurgischen Anwendung kann eine Unterdruckbefestigungseinrichtung, z.B. ein Saugring, wie er aus der WO 03/002008 A1 oder aus der EP 1 159 986 A2 bekannt ist, zum Einsatz kommen.

Die im Strahlengang des Adapters vorgesehenen Markierungsstrukturen erlauben es der Laserbearbeitungsvorrichtung, Informationen über den verwendeten Adapter zu erhalten. Dabei kann es sich beispielsweise um eine Typnummer des Adapters, eine individuelle Bezeichnung oder Informationen über die Soll-Form, die der Adapter der verformbaren Oberfläche gibt, handeln.

Um diese Information der Laserbearbeitungsvorrichtung unmittelbar bereitzustellen, sind die Markierungsstrukturen im Strahlengang so angeordnet, daß sie mittels der gescannten Laserstrahlung optisch detektierbar sind. Separate Informationsübertragungsmechanismen können dadurch entfallen, statt dessen kann die Laserbearbeitungsvorrichtung mittels geeigneter Laserstrahlabtastung die Markierungsstrukturen detektieren und damit die den Adapter kennzeichnende Information extrahieren.

Der erfindungsgemäße Adapter sowie die erfindungsgemäße Laserbearbeitungsvorrichtung ermöglichen es, applikationsangepaßte Adaptoren zu verwenden, ohne dabei Gefahr zu laufen, irrtümlich mit nicht für den aktuellen Adapter passenden Betriebsparametern zu arbeiten. Eine mögliche Fehlerquelle ist damit ausgeschaltet und insgesamt die Laserbearbeitungsqualität gesteigert.

Zweckmäßigerweise wird man bemüht sein, in der Laserbearbeitungsvorrichtung so wenig Laserstrahlquellen wie möglich einzusetzen, da zusätzliche Laserstrahlquellen üblicherweise mit weiteren Kosten verbunden sind. Man kann also daran denken, den vom Behandlungslaser abgegebenen Laserstrahl auch gleich zum optischen Detektieren der Referenzstruktur zu verwenden. Dabei muß aber die Spitzenintensität und die mittlere Leistung verringert werden, um einerseits eine Belastung am zu bearbeitenden Objekt, d.h. am Auge des Patienten zu vermeiden, und um vor allem einen Bearbeitungseffekt am Adapter zu verhindern. Es ist deshalb zweckmäßigerweise in der Laserbearbeitungsvorrichtung eine Einrichtung zur Laserstrahlenergieminderung vorzusehen, die für die optische Detektion der Referenzstruktur die Energie des vom Behandlungslaser abgegebenen Laserstrahls zumindest zeitweise mindert. Für diesen Zweck kann beispielsweise ein Energieminderer in den Strahlengang geschaltet oder im Strahlengang aktiviert werden. Alternativ kann auch die Eigenschaft üblicher gepulster Laser ausgenutzt werden, zwischen den einzelnen Laserpulsen Hintergrundstrahlung mit stark reduzierter Leistung abzugeben. Diese Hintergrundstrahlung kann für die Detektion der Referenzstruktur verwendet werden, und ein Energieminderer entfällt dann.

An welcher Stelle im Strahlengang die Referenzstruktur liegt, ist für den erfindungsgemäßen Adapter nicht ausschlaggebend; wesentlich ist lediglich, daß sie mittels von der Laserbearbeitungsvorrichtung abgegebener gescannter Laserstrahlung abtastbar sind; sie befinden sich also innerhalb eines Raumgebietes, in welchem die Laserbearbeitungsvorrichtung den Fokus des Laserstrahls positionieren kann.

Die dabei verwendete Laserstrahlung kann identisch mit der Bearbeitungslaserstrahlung sein; dies muß aber nicht der Fall sein. Zweckmäßigerweise wird jedoch die Laserbearbeitungsvorrichtung zum Scannen der Laserstrahlung die gleiche Scaneinrichtung einsetzen, die auch für die Bearbeitungslaserstrahlung verwendet wird. Das Gebiet, in dem die Referenzstruktur dann liegt, stellt also das potentielle Bearbeitungsgebiet dar.

Als Markierungsstruktur ist jegliche Ausbildung des Adapters geeignet, die es ermöglicht Information innerhalb des Strahlengangs abrufbar zu hinterlegen. Hier eignet sich die bereits genannte Gestaltung von räumlichen Zonen. Die Markierungsstrukturen können dann beispielsweise ähnlich einem Strichcode gestaltet werden, wobei eine Rückreflexion, Streuung oder Absorption bzw. Dispersion der Strahlung eine räumliche Zone auszeichnen kann.

Für die die Referenzstruktur unterscheidenden optischen Eigenschaften kommt insbesondere ein Spektralbereich in Frage, der oberhalb UV-Absorptionsbanden optischer Materialien, d.h. oberhalb von 400 nm liegt. Eine mögliche obere Grenze ergibt sich aus der angestrebten Ortsauflösung und kann typischerweise bei 2 µm angegeben werden. Vorzugsweise wird für die optische Eigenschaft ein Spektralbereich zwischen 0.8 µm und 1,1 µm genutzt. Je nach Ortsauflösung kann die Referenzstruktur Größenabmessungen zwischen 1 µm und 100 µm, vorzugsweise zwischen 3 µm und 10 µm haben.

Die durch die Markierungsstrukturen kodierte Information kann beliebige Merkmale des Adapters kennzeichnen, beispielsweise geometrische oder Material-Eigenschaften. Eine besonders bevorzugte Anwendung ist darin zu sehen, die Soll-Form, die durch die Ausgangsseite des Adapters festgelegt ist, zu beschreiben bzw. Informationen darüber zu hinterlegen. Beispielsweise können die Markierungsstrukturen die Brechungseigenschaften der Soll-Form wiedergeben.

Eine zweckmäßige Anwendung für den Adapter ist, wie bereits erwähnt, die Ausbildung als Kontaktglas für die Augenchirurgie. Es kann sich natürlich auch um ein spezielles Zubehör handeln, das das eigentliche Kontaktglas ersetzt oder zum Zwecke einer Messung vor der Behandlung am eigentlichen Kontaktglas befestigt wird.

Die erfindungsgemäße Laserbearbeitungsvorrichtung für den erfindungsgemäßen Adapter ist in einer Variante in der Lage, mittels der Detektoreinrichtung die Referenzstruktur optisch zu detektieren und in der Steuereinrichtung für die Ansteuerung der Strahlablenkungseinrichtung zu berücksichtigen. Ist die Laserbearbeitungsvorrichtung für das LASIK-Verfahren ausgebildet, kann die Steuereinrichtung die durch die Information identifizierte Soll-Form bei der Ansteuerung so berücksichtigen, daß die zu erzeugenden Durchbrüche auch an den gewünschten Stellen liegen.

Die Erfindung wird nachfolgend unter Bezugnahme auf die Zeichnung beispielhalber noch näher erläutert. In der Zeichnung zeigt:
- Fig. 1: eine schematische Darstellung einer Laserbearbeitungsvorrichtung für ein augenchirurgisches Verfahren,
- Fig. 2: eine schematische Darstellung der Augenhornhaut eines Patienten,
- Fig. 3: eine perspektivische Darstellung eines Kontaktglases für die Laserbearbeitungsvorrichtung der Figur 1,
- Fig. 4: eine Schnittdarstellung des Kontaktglases der Figur 3,
- Fig. 5: eine Draufsicht auf das Kontaktglas der Figur 3,
- Fig. 6: eine schematische Darstellung der optischen Detektion einer Referenzstruktur des Kontaktglases,
- Fig. 7: eine perspektivische Darstellung eines erfindungsgemäßen Kontaktglases für die Laserbearbeitungsvorrichtung der Figur 1,
- Fig. 8: eine Schnittdarstellung des Kontaktglases der Figur 7 und
- Fig. 9: eine Draufsicht auf das Kontaktglas der Figur 7.

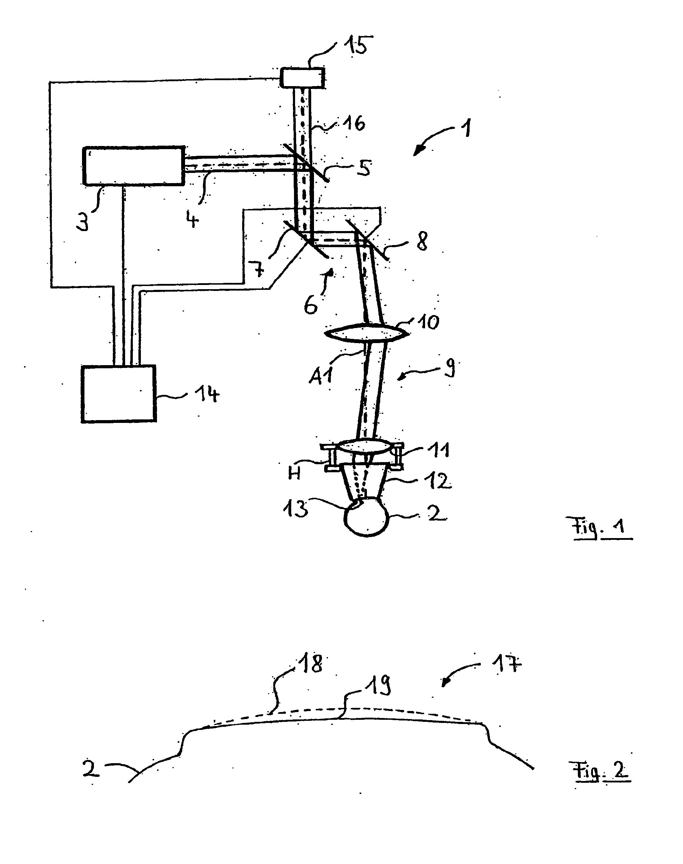

Figur 1 zeigt ein Behandlungsgerät für ein augenchirurgisches Verfahren ähnlich dem in der EP 1159986 A1 bzw. der US 5549632 beschriebenen. Das Behandlungsgerät 1 der Figur 1 dient dazu, an einem Auge 2 eines Patienten eine Fehlsichtigkeitskorrektur gemäß dem bekannten LASIK-Verfahren auszuführen. Dazu weist das Behandlungsgerät 1 einen Laser 3 auf, der gepulste Laser-Strahlung abgibt. Die Pulsdauer liegt dabei z.B. im Femtosekundenbereich, und die Laserstrahlung wirkt mittels nichtlinearer optischer Effekte in der Hornhaut auf die eingangs beschriebene Art und Weise. Der vom Laser 3 entlang einer optischen Achse A1 abgegebene Behandlungsstrahl 4 fällt dabei auf einen Strahlteiler 5, der den Behandlungsstrahl 4 auf eine Scaneinrichtung 6 leitet. Die Scaneinrichtung 6 weist zwei Scanspiegel 7 und 8 auf, die um zueinander orthogonale Achsen drehbar sind, so daß die Scaneinrichtung 6 den Behandlungsstrahl 4 zweidimensional ablenkt. Eine verstellbare Projektionsoptik 9 fokussiert den Behandlungsstrahl 4 auf bzw. in das Auge 2. Die Projektionsoptik 9 weist dabei zwei Linsen 10 und 11 auf.

Der Linse 11 ist ein Kontaktglas 12 nachgeordnet, das über eine Halterung H fest mit der Linse 11 und damit dem Strahlengang des Behandlungsgerätes 1 verbunden ist. Das noch näher zu beschreibende Kontaktglas 12 liegt an der Hornhaut des Auges 2 an. Die optische Kombination aus Behandlungsgerät 1 mit daran befestigtem Kontaktglas 12 bewirkt, daß der Behandlungsstrahl 4 in einem in der Hornhaut des Auges 2 gelegenen Fokus 13 gebündelt wird.

Die Scaneinrichtung 6 wird ebenso wie der Laser 3 und die Projektionsoptik 9 über (nicht näher bezeichnete) Steuerleitungen von einem Steuergerät 14 angesteuert. Das Steuergerät 14 bestimmt dabei die Lage des Fokus 13 sowohl quer zur optischen Achse A1 (durch die Scanspiegel 7 und 8) sowie in Richtung der optischen Achse A1 (durch die Projektionsoptik 9) vor.

Das Steuergerät 14 liest weiter einen Detektor 15 aus, der von der Hornhaut rückgestreute Strahlung, die den Strahlteiler 5 als Rückstrahlung 16 passiert, ausliest. Dazu kann eine konfokale Abbildung erfolgen. Auf die Bedeutung des Detektors 15 wird später noch eingegangen.

Das Kontaktglas 12 sorgt dabei dafür, daß die Hornhaut des Auges 2 eine gewünschte Soll-Form erhält. Das Auge 2 befindet sich aufgrund der Anlage der Hornhaut 17 am Kontaktglas 12 in vorbestimmter Lage zum Kontaktglas 12 und damit zum damit verbundenen Behandlungsgerät 1.

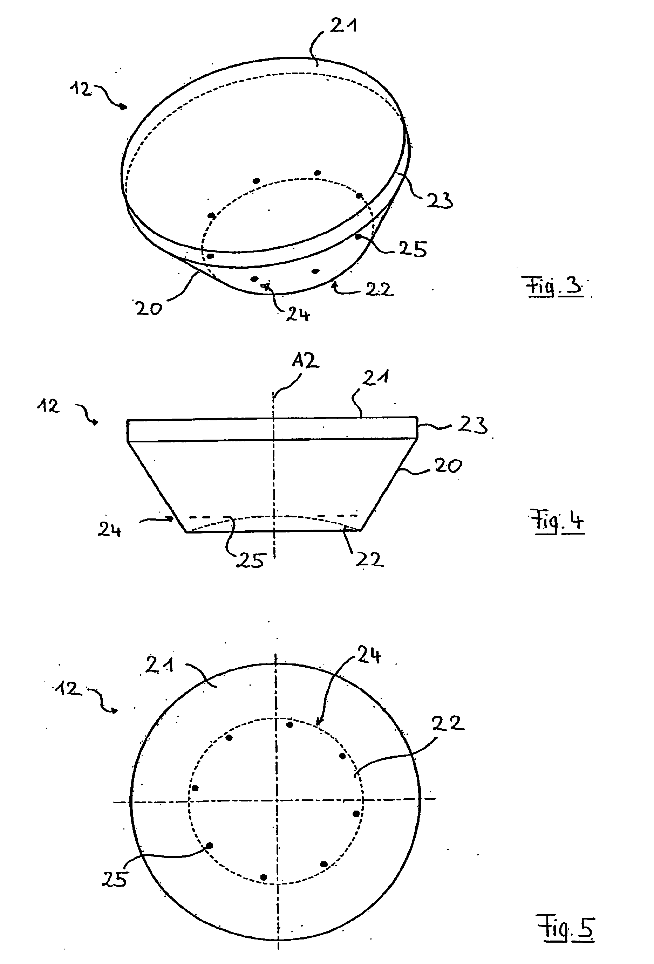

Dies ist schematisch in Figur 2 dargestellt, die einen Schnitt durch die Augenhornhaut 17 zeigt. Um eine exakte Positionierung des Fokus 13 in der Augenhornhaut 17 zu erreichen, muß die Krümmung der Augenhornhaut 17 berücksichtigt werden. Die Augenhornhaut 17 weist dabei eine Ist-Form 18 auf, die von Patient zu Patient unterschiedlich ist. Das Kontaktglas 12 liegt nun an der Augenhornhaut 17 derart an, daß es diese in eine gewünschte Soll-Form 19 verformt. Der genaue Verlauf der Soll-Form 19 hängt dabei von der Krümmung der dem Auge 2 zugewandten Fläche des Kontaktglases ab. Dies wird später noch anhand der Figur 4 deutlicher werden. Wesentlich ist hier lediglich, daß durch das Kontaktglas 12 bekannte geometrische und optische Verhältnisse für das Einbringen und Fokussieren des Behandlungsstrahls 4 in die Hornhaut 17 gegeben sind. Da die Hornhaut 17 am Kontaktglas 12 anliegt und dieses wiederum über die Halterung H gegenüber dem Strahlengang des Behandlungsgerätes 1 ortsfest ist, kann der Fokus 13 durch Ansteuerung der Scaneinrichtung 6 sowie der verstellbaren Projektionsoptik 9 dreidimensional exakt in der Hornhaut 17 positioniert werden.

Figur 3 zeigt eine perspektivische Darstellung des Kontaktglases 12. Wie zu sehen ist, weist das Kontaktglas 12 einen Glaskörper 20 auf, der für den Behandlungsstrahl 4 transparent ist. An einer Oberseite 21 des kegelstumpfartigen Glaskörpers 20 wird der Behandlungsstrahl 4 eingekoppelt; diese Oberseite 21 ist der Linse 11 zugeordnet.

An einer Unterseite 22 des Kontaktglases 12 liegt die Hornhaut 17 an. Wie die Schnittdarstellung der Figur 4 zeigt, ist die Unterseite 22 in der gewünschten Soll-Form 19 gekrümmt, so daß sie bei voller Kontaktierung des Auges 2 die gewünschte Form der Hornhaut 17 bewirkt.

Nahe der Oberseite 21 ist am Kontaktglas 12 eine Flanschfläche 23 ausgebildet, an der das Kontaktglas 12 in der Halterung H durch Klemmen fixiert ist. Die Flanschfläche 23 stellt ein Befestigungsmittel dar, das auf die einen Verschlußmechanismus realisierende Halterung H abgestimmt ist.

Die Hauptsymmetrieachse A2 des kegelstumpfförmigen Glaskörpers 20 wird durch Befestigung über die Flanschfläche 23 in fester Verbindung mit dem Behandlungsgerät 21 und passend zur optischen Achse A1 einjustiert. Im Inneren des Glaskörpers 20 ist eine Referenzstruktur 24, im Ausführungsbeispiel ringförmig, gebildet. Der Abstand zur optischen Hauptachse A2 ist im Ausführungsbeispiel möglichst groß gewählt, so daß die Referenzstruktur 24 nur bei nahezu maximal ausgelenkten Behandlungsstrahl 4 im vom Behandlungsstrahl 4 durchstrahlten Volumen des Glaskörpers 20 liegt.

Wie die Figuren 4 und 3 zeigen, liegt die Referenzstruktur 24 im Volumen des Glaskörpers 20 vorzugsweise am Rand bzw. nahe des Randes des kegelstumpfartigen Glaskörpers 20. Die Referenzstruktur 24 besteht aus mehreren Reflektorzonen 25, die für die vom Laser 3 abgegebene Strahlung reflektierend sind.

Die Reflektorzone 25 kann auch auf der Oberseite 21 oder der Unterseite 22 des Kontaktglases 12, d. h. auf der Eintritts- oder Austrittsfläche des Adapters in Form einer geeigneten Schichtstruktur oder geeigneter reflektierender oder nicht-reflektierende Schichten aufgebracht werden. Auch ist es möglich, Zonen oder Schichten mit erhöhter elastischer Lichtstreuung vorzusehen, um die Reflektorzonen 25 zu realisieren.

Fällt der Behandlungsstrahl 4 auf eine Reflektorzone 25, wird Strahlungsenergie zurückgestreut, die dann vom Detektor 15 aufgenommen wird. Anhand des Signals des Detektors 15 kann das Steuergerät 14 somit erkennen, ob der Behandlungsstrahl 4 auf eine Reflektorzone 25 gerichtet ist.

Wie in Figur 5 zu sehen ist, liegen die Reflektorzonen 25 ringförmig nahe des Randes der Unterseite 22. Die Unterseite 22 gibt zusammen mit der durch die Scaneinrichtung 6 möglichen Ablenkung die Größe und Lage des Bearbeitungsgebietes vor. Bei einer Fehlpositionierung des Bearbeitungsgebietes auf der Augenhornhaut 17 kommt es zu einer Abweichung zwischen einem gewünschten und einem erzielten Refraktionsergebnis, so daß eine angestrebte Fehlsichtigkeitskorrektur mitunter nicht erreicht werden kann. Die Reflektorzonen 25 dienen dazu, die tatsächliche Strahlablenkung mit einem vorgegebenen Soll-Wert zu vergleichen und dadurch Bearbeitungsfehler zu minimieren.

Abweichungen zwischen tatsächlicher Strahlposition und vorgegebener Soll-Lage auf der Augenhornhaut 17 können prinzipiell durch Bewegungen des Auges relativ zum Behandlungsgerät 1 oder durch eine Fehlpositionierung des Auges 2 gegenüber dem Behandlungsgerät 1 oder durch eine Fehlpositionierung der Scanspiegel 7, 8 sowie der Projektionsoptik 9 hervorgerufen werden. Das Kontaktglas 12 bewirkt eine fixe Positionierung des Auges 2 gegenüber dem Behandlungsgerät 1, da die Augenhornhaut 17 über geeignete Mittel, beispielsweise einen (nicht näher dargestellten) Saugring, am Auge 2 fixiert wird. Die Reflektorzonen 25 dienen nun dazu, die Lage des Auges 2 gegenüber dem Behandlungsgerät 1 ermitteln zu können.

Das Steuergerät 14 steuert die Scaneinrichtung 6 sowie die Projektionsoptik 9 so an, daß ein Laserstrahl über die Reflektorzonen 25 geführt wird. Beispielsweise steuert das Steuergerät 14 den Laser 3 in einen Betriebsmodus, in dem nur ein Strahl 4 mit stark verringerter Strahlungsintensität abgegeben wird. Dies kann beispielsweise durch Aktivieren oder Einschwenken eines geeigneten Strahlungsabschwächers erfolgen. Handelt es sich beim Laser 3 um eine gepulste Laserstrahlungsquelle kann auch eine außerhalb des Pulsbetriebes eventuell vorliegende, sehr viel schwächere Hintergrundstrahlung verwendet werden. Alternativ ist es möglich, einen zusätzlichen Laser einzukoppeln, beispielsweise über einen weiteren Strahlteiler, der der Scaneinrichtung 3 vorgeordnet ist. Bei diesem Laserstrahl kann es sich also entweder um den gegebenenfalls geeignet abgeschwächten Behandlungsstrahl 4 oder um einen separaten Laserstrahl handeln, der vor der Scaneinrichtung 6 in den Strahlengang entlang der optischen Achse A1 eingekoppelt wird.

Trifft der Laserstrahl auf eine Reflektorzone 25, zeigt der Detektor 15 ein entsprechendes Signal an. Wird eine Reflektorzone 25 so detektiert, speichert das Steuergerät 14 die dabei gegebenen Einstellwerte für die Scaneinrichtung 6 sowie die Projektionsoptik 9 ab. Nach Abtasten von mindestens drei Reflektorzonen 25 ist damit eine vollständige Bestimmung der tatsächlichen Ist-Lage des Kontaktglases 12 und damit der Augenhornhaut 17 erreicht. Diese Ist-Lage verwendet das Steuergerät 14, um in der nachfolgenden Behandlung mit dem Behandlungsstrahl 4 den Fokus 13 an gewünschten vorgegebenen Stellen in der Augenhornhaut 17 zu plazieren.

Durch die ringförmige Referenzstruktur 24 am Rande des Bearbeitungsgebietes ist eine ungestörte Behandlung im Zentrum der durch die Unterseite 22 umschriebenen Querschnittsfläche, durch die der Behandlungslaserstrahl 4 in die Augenhornhaut 17 eingekoppelt wird, möglich. Durch eine ausreichend große numerische Apertur der Behandlungsstrahlung ist der Einfluß der im Randbereich des Bearbeitungsgebietes liegenden Reflektorzonen 25 bei der Behandlung vernachlässigbar.

Die Lage der Reflektorzonen 25 am Rand der Unterseite 22 erlaubt es, im laufenden Betrieb die Funktion der Scaneinrichtung 6 sowie der Projektionsoptik 9 zu überprüfen. Dabei kann eine relative Abweichung zwischen gespeicherter Ist-Lage der Reflektorzonen 25 sowie bei einer erneuter Überprüfung zugeordneter Einstellungen der Scaneinrichtung 6 sowie Projektionsoptik 9 erfolgen, um im Betrieb auftretende Abweichungen auskorrigieren zu können oder gegebenenfalls den Betrieb des Behandlungsgerätes 1 zu sperren, falls eine zu große Abweichung vorliegt.

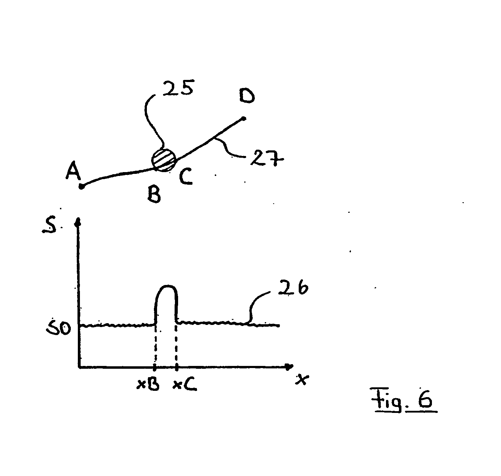

Den Vorgang der Detektion einer Reflektorzone 25 veranschaulicht Figur 6. Dort ist ein Signal S des optischen Detektors 15 als Kurve 26 eingetragen. Der Fokus 13 wird auf einer Bahn 27, die im Regelfall dreidimensional ausgebildet, in Figur 2 jedoch nur zweidimensional dargestellt ist, von einem Punkt A zu einem Punkt D geführt, der den Bereich, in dem eine Reflektorzone 25 erwartet wird, überdeckt. Während der Bewegung des Laserfokus 13 vom Punkt A liefert der Detektor 15 einen Ruhewert S0. Beim Erreichen des Punktes B ändert sich das Signal und steigt an, da ein Rückreflex an der Reflektorzone 25 eintritt. Die zugehörige Koordinate xB in x-Richtung (in Figur 6 ist das Signal S nur eindimensional bezüglich der x-Richtung eingetragen) kennzeichnet den Beginn der Reflektorzone 25 in x-Richtung.

Mit Erreichen des Punktes C sinkt das Signal wieder auf den Ruhewert S0, und die Koordinate xC bezeichnet das Ende der Reflektorzone in x-Richtung. Ist der Durchmesser des Fokus 13 klein gegen die Ausdehnung der Reflektorzone 25 und damit klein gegen die Entfernung BC, ist die in Figur 6 dargestellte deutliche Trennung der steigenden Flanke bei xB und der fallenden Flanke bei xC möglich. In diesem Fall kann die gewonnene Information über die Lage dieser Koordinaten bei der Bestimmung der Position der Reflektorzone 25 mit im Steuergerät 14 berücksichtigt werden, wenn die Reflektorzone 25 eine bekannte Form hat. Ist der Durchmesser des Laserfokus 13 dagegen gleich oder größer dem Abstand BC, lassen sich die Koordinaten xB und xC nicht unterscheiden, und das Zentrum der Reflektorzone 25 erscheint im Signal S.

Die in Figur 2 eindimensional geschilderte Ortsbestimmung durch optisches Abtasten erfolgt natürlich in drei Raumkoordinaten, so daß die Lage der Reflektorzone 25 insgesamt dreidimensional bestimmt wird.

Die Detektion der Reflektorzone 25 im Bearbeitungsgerät der Figur 1 kann vorzugsweise konfokal erfolgen, um eine möglichst hohe Auflösung entlang der optischen Achse A1 bzw. A2 (d. h. in Tiefenrichtung) zu erzielen.

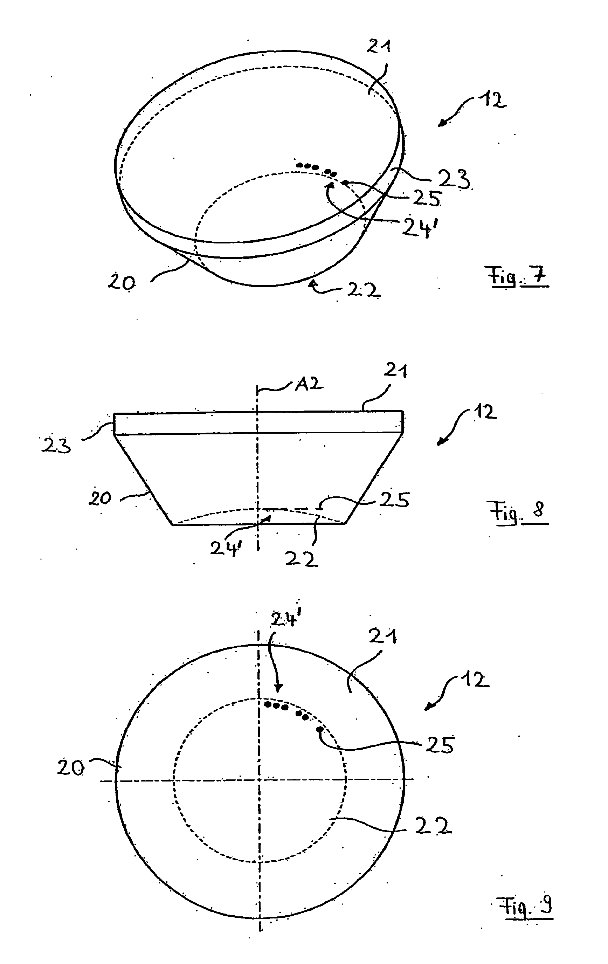

Die Figuren 7 bis 9 zeigen einen Adapter, der wie der der Figuren 3 bis 5 als Kontaktglas 12 ausgebildet ist, sich jedoch hinsichtlich der erfindungsgemäßen Ausbildung der Referenzstruktur unterscheidet. Aufgrund der ansonsten gegebenen Übereinstimmungen wird auf die Beschreibung der Figuren 3 bis 5 verwiesen, und für gleiche Merkmale werden die gleichen Bezugszeichen verwendet.

Im Inneren des Glaskörpers 20 ist nun als Referenzstruktur eine Codestruktur 24', im Ausführungsbeispiel ringförmig, gebildet. Der Abstand zur optischen Hauptachse A2 ist im Ausführungsbeispiel möglichst groß gewählt, so daß die Codestruktur 24' nur bei nahezu maximal ausgelenkten Behandlungsstrahl 4 im vom Behandlungsstrahl 4 durchstrahlten Volumen des Glaskörpers 20 liegt.

Wie die Figuren 7 und 9 zeigen, liegt die Codestruktur 24' im Volumen des Glaskörpers vorzugsweise am Rand bzw. nahe des Randes des kegelstumpfartigen Glaskörpers 20. Die Codestruktur 24' besteht aus mehreren Reflektorzonen 25, die für die vom Laser 3 abgegebene Strahlung reflektierend sind. Fällt der Behandlungsstrahl 4 auf eine Reflektorzone 25, wird Strahlungsenergie zurückgestreut, die dann vom Detektor 15 aufgenommen wird.

Die Reflektorzone 25 kann auch auf der Oberseite 21 oder der Unterseite 22 des Kontaktglases 12, d. h. auf der Eintritts- oder Austrittsfläche des Adapters in Form einer geeigneten Schichtstruktur oder geeigneter reflektierender oder nicht-reflektierende Schichten aufgebracht werden. Auch ist es möglich, Zonen oder Schichten mit erhöhter elastischer Lichtstreuung vorzusehen, um die Reflektorzonen 25 zu realisieren.

Anhand des Signals des Detektors 15 kann das Steuergerät 14 somit erkennen, daß der Behandlungsstrahl 4 auf eine Reflektorzone 25 gerichtet ist. Die Abfolge der ringförmig angeordneten Reflektorzonen 25 in der Codestruktur 24' liefert damit insgesamt ein codiertes Signal, das im Ausführungsbeispiel die Krümmung der Unterseite 22 des Glaskörpers 20 und damit die Geometrie der Soll-Form 19 wiedergibt, die die Augenhornhaut 17 mit dem aufgelegten Kontaktglas 12 hat. Die Codestruktur 24' realisiert somit Markierungsstrukturen, die das Kontaktglas 12 identifizieren oder beschreiben.

Um diese prinzipiell bereits erläuterte Informationsextraktion durchzuführen, steuert das Steuergerät 14 zum einen den Laser 3 in einen Betriebsmodus, in dem nur ein Strahl 4 mit stark verringerter Strahlungsintensität abgegeben wird. Dies kann beispielsweise durch Aktivieren oder Einschwenken eines geeigneten Strahlungsabschwächers erfolgen. Handelt es sich beim Laser 3 um eine gepulste Laserstrahlungsquelle kann auch eine außerhalb des Pulsbetriebes eventuell vorliegende, sehr viel schwächere Hintergrundstrahlung verwendet werden.

Alternativ ist es möglich, einen zusätzlichen Laser einzukoppeln. beispielsweise über einen weiteren Strahlteiler, der der Scaneinrichtung 3 vorgeordnet ist. Bei diesem Laserstrahl kann es sich also entweder um den gegebenenfalls geeignet abgeschwächten Behandlungsstrahl 4 oder um einen separaten Laserstrahl handeln, der vor der Scaneinrichtung 6 in den Strahlengang entlang der optischen Achse A1 eingekoppelt wird.

Um die Codestruktur 24' auszulesen, steuert das Steuergerät 14 die Projektionsoptik 9 sowie die Scaneinrichtung 6 derart an, daß der Fokus der Laserstrahlung über den Bereich geführt wird, in dem die Codestruktur 24' erwartet wird. Die Rückreflexe werden im Signal des Detektors 15 erkannt, der aktuellen Fokuslage zugeordnet und mit Hilfe geeigneter Mittel, (beispielsweise geeigneter Verarbeitungselektronik und eines Speicherbausteins) hinsichtlich der codierten Information bewertet. Die Detektion der Reflektorzone 25 im Bearbeitungsgerät der Figur 1 kann vorzugsweise konfokal erfolgen, um eine möglichst hohe Auflösung entlang der optischen Achse A1 bzw. A2 (d. h. in Tiefenrichtung) zu erzielen.

Die derart gewonnene Information über den Adapter wird dann vom Steuergerät 14 bei der nachfolgenden Behandlung der Augenhornhaut 17 berücksichtigt. Beispielsweise wird die Steuerung des Fokus 13 mittels der Scaneinrichtung 6 und der Projektionsoptik 9 so bewirkt, daß die Soll-Form 19 des aktuell verwendeten Kontaktglases 12 berücksichtigt wird. Alternativ kann auch beim Scannen eines untauglichen Kontaktglases das Behandlungsgerät 1 gesperrt werden, so daß keine Behandlung möglich ist. Zusätzlich oder alternativ kann eine entsprechende Information über das aktuell verwendete Kontaktglas mit geeigneten Mitteln ausgegeben werden.

## Patentansprüche

1. Adapter zum Koppeln einer Laserbearbeitungsvorrichtung (1) mit einem zu bearbeitenden Objekt (2), das eine verformbare Oberfläche aufweist, wobei der Adapter (12)
- eine Eingangsseite (21) aufweist, die über einen Verschlußmechanismus (H) gegenüber der Laserbearbeitungsvorrichtung (1) fixierbar ist,
- zur Ausrichtung des Objektes (2) gegenüber der Laserbearbeitungsvorrichtung (1) am Objekt (2) befestigbar ist,
- einen von der Laserbearbeitungsvorrichtung (1) über einen gewissen Bereich gescannt an der Eingangsseite (21) zugeführten Laserstrahl (4) über einen Strahlengang zum Objekt (2) leitet und
- eine Referenzstruktur (24) aufweist, die im Strahlengang des Adapters (12) liegt und mittels der über den Bereich gescannten Laserstrahlung (4) optisch detektierbar ist, und
- eine Ausgangsseite (22) aufweist, die an die verformbare Oberfläche (17) anlegbar ist und dieser dabei eine gewünschte Soll-Form (19) gibt, **dadurch gekennzeichnet, daß** die Referenzstruktur als Markierungsstrukturen (25) ausgebildet ist, die eine den Adapter (12) kennzeichnende Information codieren.

2. Adapter nach Anspruch 1, **dadurch gekennzeichnet, daß** die Referenzstruktur räumliche Zonen (25) aufweist, die sich mindestens hinsichtlich einer optischen Eigenschaft vom restlichen Strahlengang des Adapters (12) unterscheiden.

3. Adapter nach Anspruch 2, **dadurch gekennzeichnet, daß** die optische Eigenschaft der Brechungsindex ist.

4. Adapter nach einem der obigen Ansprüche, **dadurch gekennzeichnet, daß** der Strahlengang zumindest teilweise ein für Bearbeitungslaserstrahlung transparentes Material (20), insbesondere Glas, aufweist.

5. Adapter nach Anspruch 4, **gekennzeichnet durch** einen zylindrischen oder kegelstumpfartigen Körper, dessen eine Endfläche (22) als Ausgangsseite mit der gewünschten Soll-Form (19) der Oberfläche (17) ausgebildet ist.

6. Adapter nach Anspruch 4, **gekennzeichnet durch** einen zylindrischen oder kegelstumpfartigen Körper (20), dessen eine Endfläche (21) als Eingangsseite ausgebildet ist.

7. Adapter nach einem der obigen Ansprüche, **gekennzeichnet durch** einen Flansch (23) für den Verschlußmechanismus (H).

8. Adapter nach einem der obigen Ansprüche, **gekennzeichnet durch** eine Unterdruckbefestigungseinrichtung zum Befestigen am Objekt (2).

9. Adapter nach einem der obigen Ansprüche, **dadurch gekennzeichnet, daß** die Information die durch die Ausgangsseite (22) festgelegte Soll-Form (19) kennzeichnet, insbesondere deren Brechungseigenschaften.

10. Adapter nach einem der obigen Ansprüche, **dadurch gekennzeichnet, daß** er als Kontaktglas für die Augenchirurgie ausgebildet ist.

11. Laserbearbeitungsvorrichtung für einen Adapter nach einem der obigen Ansprüche, mit
- einer Strahlablenkungseinrichtung (6, 9) zum Scannen eines Laserstrahls (4) über den gewissen Bereich,
- einer Detektoreinrichtung (5, 15) zum optischen Detektieren der Markierungsstrukturen (25) mittels des Laserstrahls (4) und
- einer die Detektoreinrichtung (5, 15) auslesende Steuereinrichtung (14), welche die Strahlablenkungseinrichtung (6) ansteuert, die den Adapter (12) kennzeichnende Information ermittelt und diese bei der Ansteuerung der Strahlablenkungseinrichtung (6, 9) berücksichtigt.

12. Laserbearbeitungsvorrichtung nach Anspruch 11, **gekennzeichnet durch** einen gepulsten Behandlungslaser (3) für ein augenchirurgisches Verfahren, bei dem das Objekt die Augenhornhaut (17) ist, wobei die Steuereinrichtung (14) die Strahlablenkungseinrichtung (6, 9) und den Behandlungslaser (3) so ansteuert, daß der Laserstrahl (4) an vorbestimmten Stellen (13) im Auge (2) optische Durchbrüche erzeugt, und dabei die **durch** die Information identifizierte Soll-Form (19) der Oberfläche der Augenhornhaut (17) berücksichtigt.

13. Laserbearbeitungsvorrichtung nach Anspruch 11, **gekennzeichnet durch** einen gepulsten Behandlungslaser (3) für ein augenchirurgisches Verfahren und eine Einrichtung zur Laserstrahlenenergieminderung, die zum optischen Detektieren der Referenzstruktur (25) die Energie des vom Behandlungslaser (3) abgegebenen Laserstrahls (4) zumindest zeitweise mindert.

## Claims

1. An adapter for coupling a laser treatment device (1) with an object (2) to be processed, the object comprising a deformable surface, wherein the adapter (12)
- comprises an input side (21), which can be fixated relative to the laser processing device (1) via a locking mechanism (H);
- is attachable to the object (2) for positioning of the object (2) relative to the laser processing device (1);
- transmits a laser beam (4) to the object (2) along a beam path, said laser beam (4) having been supplied to the adapter input side (21) by the laser processing device (1) and scanned over a certain region, and
- comprises a reference structure (24), which is located in the adapter beam path and is optically detectable by means of the laser radiation (4) scanned across said region,
- comprises an output side (22) which can be brought into contact with the deformable surface (17) and thereby imparts a desired shape (19) to said surface (17),
**characterized in that** the reference structure is provided as marking structures (25) which encode information characterizing the adapter (12)..

2. The adapter as claimed in Claim 1, **characterized in that** the reference structure (24) comprises spatial zones (25), which differ from the remaining beam path of the adapter (12) in at least one optical property.

3. The adapter as claimed in Claim 2, **characterized in that** the optical property is the refractive index.

4. The adapter as claimed in any one of the above Claims, **characterized in that** the beam path at least partially comprises a material (20), in particular glass, which is transparent for the processing laser radiation.

5. The adapter as claimed in Claim 4, **characterized by** a cylindrical or frustoconical body, one end surface (22) of which is provided as the output side having the desired shape (19) of the surface (17).

6. The adapter as claimed in Claim 4, **characterized by** a cylindrical or frustoconical body (20), one end surface (21) of which is provided as the input side.

7. The adapter as claimed in any one of the above Claims, **characterized by** a flange (23) for the locking mechanism (H).

8. The adapter as claimed in any one of the above Claims, **characterized by** a suction attachment unit for attachment to the object (2).

9. The adapter as claimed in any one of the above Claims, **characterized in that** the information characterizes the desired shape (19) defined by the output side (22), in particular the refractive properties thereof.

10. The adapter as claimed in any one of the above Claims, **characterized in that** it is provided as a contact glass for eye surgery.

11. A laser processing device for an adapter as claimed in any one of the above Claims, comprising
- a beam deflecting unit (6, 9) for scanning a laser beam (4) over the certain region,
- a detecting unit (5, 15) for optical detection of the marking structures (25) by means of the laser beam (4), and
- a control unit (14) which reads out the detecting unit (5, 15), controls the beam deflecting unit (6), determines the information characterizing the adapter (12) and considers said information when controlling the beam deflecting unit (6, 9).

12. The laser processing device as claimed in Claim 11, **characterized by** a pulsed treatment laser (3) for an ophthalmic surgery procedure, wherein the object is the cornea (17) of an eye, with the control unit (14) controlling the beam deflecting unit (6, 9) and the treatment laser (3) such that the laser beam (4) generates optical breakthroughs at predetermined locations (13) in the eye (2) and, in doing so, considers the desired shape (19) of the surface of the cornea (17) of the eye, which shape (19) is identified by said information.

13. The laser processing device as claimed in Claim 11, **characterized by** a pulsed treatment laser (3) for an ophthalmic surgery procedure and by a means for reducing the laser beam energy, which means at least temporarily reduces the energy of the laser beam (4) emitted by the treatment laser (3) for optical detection of the reference structure (25).

## Revendications

1. Adaptateur pour accoupler un dispositif (1) de traitement au laser à un objet (2) à traiter qui présente une surface déformable, sachant que l'adaptateur (12)
- présente un côté d'entrée (21), qui peut être immobilisé par rapport au dispositif (1) de traitement au laser au moyen d'un mécanisme de verrouillage (H),
- peut être fixé sur l'objet (2) afin de positionner l'objet (2) par rapport au dispositif (1) de traitement au laser,
- guide jusqu'à l'objet (2), via un trajet de faisceau, un faisceau laser (4) acheminé par le dispositif (1) de traitement au laser jusqu'au côté d'entrée (21) en étant balayé sur une zone donnée, et
- présente une structure de référence (24), qui se situe dans le trajet de faisceau de l'adaptateur (12) et qui peut être détectée optiquement au moyen du faisceau laser (4) balayé sur la zone,
- présente un côté de sortie (22), qui peut être appliqué contre la surface déformable (17) et confère alors à celle-ci une forme de consigne souhaitée (19),
**caractérisé en ce que** la structure de référence est réalisée sous la forme de structures de marquage (25) qui codent une information caractérisant l'adaptateur (12).

2. Adaptateur selon la revendication 1, **caractérisé en ce que** la structure de référence présente des zones spatiales (25) qui se différencient du reste du trajet de faisceau de l'adaptateur (12) par au moins une propriété optique.

3. Adaptateur selon la revendication 2, **caractérisé en ce que** la propriété optique est l'indice de réfraction.

4. Adaptateur selon l'une des revendications précédentes, **caractérisé en ce que** le trajet de faisceau présente au moins pour partie un matériau (20) transparent au faisceau laser de traitement, en particulier du verre.

5. Adaptateur selon la revendication 4, **caractérisé par** un corps cylindrique ou tronconique, dont une face terminale (22) est conçue comme côté de sortie avec la forme de consigne souhaitée (19) de la surface (17).

6. Adaptateur selon la revendication 4, **caractérisé par** un corps cylindrique ou tronconique (20), dont une face terminale (21) est conçue comme côté d'entrée.

7. Adaptateur selon l'une des revendications précédentes, **caractérisé par** une bride (23) pour le mécanisme de verrouillage (H).

8. Adaptateur selon l'une des revendications précédentes, **caractérisé par** un dispositif de fixation à dépression pour la fixation sur l'objet (2).

9. Adaptateur selon l'une des revendications précédentes, **caractérisé en ce que** l'information caractérise la forme de consigne (19) définie par le côté de sortie (22), en particulier ses propriétés de réfraction.

10. Adaptateur selon l'une des revendications précédentes, **caractérisé en ce qu'**il est conçu comme verre de contact pour la chirurgie oculaire.

11. Dispositif de traitement au laser pour un adaptateur selon l'une des revendications précédentes, avec
- un dispositif (6, 9) de déviation de faisceau pour balayer un faisceau laser (4) sur la plage donnée,
- un dispositif détecteur (5, 15) pour la détection optique des structures de marquage (25) au moyen du faisceau laser (4), et
- un dispositif de commande (14), qui lit le dispositif détecteur (5, 15), commande le dispositif (6) de déviation de faisceau, détermine l'information caractérisant l'adaptateur (12) et en tient compte pour la commande du dispositif (6, 9) de déviation de faisceau.

12. Dispositif de traitement au laser selon la revendication 11, **caractérisé par** un laser de traitement pulsé (3) pour une opération de chirurgie oculaire où l'objet est la cornée (17) de l'oeil, sachant que le dispositif de commande (14) commande le dispositif (6, 9) de déviation de faisceau et le laser de traitement (3) de telle sorte que le faisceau laser (4) produit des percées optiques en des endroits prédéterminés (13) dans l'oeil (2), et tient alors compte de la forme de consigne (19), identifiée par ladite information, de la surface de la cornée (17).

13. Dispositif de traitement au laser selon la revendication 11, **caractérisé par** un laser de traitement pulsé (3) pour une opération de chirurgie oculaire et par un dispositif pour réduire l'énergie du faisceau laser, qui réduit au moins temporairement l'énergie du faisceau laser (4) émis par le laser de traitement (3) afin de détecter optiquement la structure de référence (25).
